# EUROPÄISCHE PATENTANMELDUNG

(11) **EP 1 852 400 A1**
(43) Veröffentlichungstag der Anmeldung: **07.11.2007**
(21) Anmeldenummer: 06009210.3
(22) Anmeldetag: 04.05.2006
(51) Int. Cl.: C02F 11/04

(54) **Verfahren zur Gewinnung von Methangas**

(71) Anmelder: Pfefferkorn, Herbert, 6900 Bregenz (AT)
(72) Erfinder: Pfefferkorn, Herbert, 6900 Bregenz (AT)

(57) **Zusammenfassung**

Die Erfindung umfasst ein Verfahren zur Gewinnung von Methangas aus organisch beladenen Substraten in gelöster und fester Form, bestehend aus einem oder mehreren Reaktor(en), vorzugsweise in einem oder mehreren Behälter(n) mit mindest je einem Zu- und Ablauf versehen.

Die Neuheit stellt dabei die Art der ganz oder teilweise in das Gärsubstrat eingetauchten Rührwerke dar, deren Antrieb über Nutzung der Auftriebskräfte der aus dem Fermentationsprozess gewonnenen Gase. Mit dem(n) kombinierten Betrieb nach dem Verdrängersystem konzipierten Reatkor(en) wird eine weitere Misch- und Rühreffizienz durch Nutzung der unterschiedlich anfallenden Gasmengen und Gasdrücke erreicht.

Die Vorteile dieser Neuentwicklung stellen vor allem die sehr einfache Konstruktion sowie der Betrieb der Rührwerke ohne Fremdenergie durch die Nutzung der Auftriebskraft der gewonnenen Gase dar. Diese Rührwerke sind auch bei großen Ausführungsarten, bedingt durch die Krafteinleitung über die Peripherie, sehr materialschonend und weisen dabei einen maximalen Wirkungsgrad auf.

## Beschreibung

Die Erfindung bezieht sich auf ein Verfahren zur Gewinnung von Methangas aus organisch beladenen Substraten in gelöster und fester Form, bestehend aus einem oder mehreren Reaktor(en), vorzugsweise in Form eines oder mehreren Behälter mit mindest je einem Zu- und Ablauf versehen, in welchen die Gärsubstratdurchmischung und Gärsubstratführung im wesentlichen über Rührwerke erfolgt, deren Antrieb über Nutzung der Auftriebskräfte des aus dem Fermentationsprozess gewonnene Biogases erfolgt. Das gewonnene Biogas wird dabei in Gasauffanghauben, welche auch gleichzeitig als Rührwerkzeuge geformt werden, eingeleitet und die in der Folge auftretende Auftriebskraft wird für den Antrieb der oder des Rührwerke(s) genützt. Durch die kombinierte Nutzung der gewonnenen und verdichteten Gase im (in) nach dem Verdrängerprinzip konzipierten Reaktor(en) wird die Rühr- und Mischeffizienz weiter erhöht. Diese Art Rührtechnik stellt eine sehr effizient arbeitende - ohne fremde Energiezufuhr - verschleißarme Lösung dar.

Folgend angeführte Einrichtungen gehören zum Stand der Technik. Die bisherigen Erfahrungen in der Praxis mit verschiedensten Arten und Typen von Biogasreaktoren zeigen vermehrt Probleme in Form von zu geringen Gasproduktionen sowie den Gärprozess behindernden Störungen durch Ansammlungen von Feststoffen in Form von mächtigen Schwimm- und

Sinkschichten in den Reaktoren. Vor allem stellt der zu hohe Aufwand von mechanischer Energie für die Substratführung und Substratdurchmischung ein effizienter Betrieb derartiger Anlagen in Frage.

Die Gärsubstratführung sowie Durchmischung werden heute im Wesentlichen nach folgenden Methoden durchgeführt:
1. Umwälzung durch den Betrieb von in den Behältern eingebauten mechanischen Rühr- und Mischeinrichtungen.
2. Umwälzung mittels Verdrängersystem.
3. Umwälzung durch extern angeordnete Pumpen.
4. Umwälzung durch Gaseinpressung in die Gärmasse.

Die heute meist angewandte Technik beim Betrieb von Biogasreaktoren stellt das Verdrängersystem dar. Bei diesem Verfahren werden in der Regel zwei oder mehrere Räume gegenseitig kommunizierend verbunden, dabei wird in einem oder mehreren dieser Räume der Gasabgang periodisch verriegelt. Der oder die restlichen Räume bleiben auf der Gasseite zum Gaslager offen. Nach Erreichen der vorgegebenen Größe des verdichteten Gaspolsters in dem oder den verriegelten Gasraum(räumen) wird das Gasabsperrorgan geöffnet, wodurch in der Folge des Druckabfalls im Gasbereich gleichzeitig eine Rückströmung der verdrängten Gärmasse erreicht wird. Mit dieser Rückströmung wird eine Durchmischung der Gärmasse erreicht.

Trotz stetiger Weiterentwicklung und Verbesserung dieser verschiedener Biogastechniken stellt heute vor allem im Bereich der Landwirtschaft die immer mehr zum Einsatz kommende Verarbeitung von Gärsubstraten in Form von frischer oder auf bestimmte Zeit konservierte Pflanzenmasse ein großes Problem dar. Soweit diese angeführten Substrate in Cofermentation mit der üblicherweise vorhandenen Gülle verarbeitet werden, ist neben den zeitlich stark unterschiedlichen mikrobiellen Ab- und Umbaugeschwindigkeiten vor allem auch dem Trend zur extremen Schwimmdeckenbildung Rechnung zu tragen. In der Praxis versucht man durch den Einsatz mit modifizierten mechanischen Misch- Rühr- und

Abscheidevorrichtungen diesen Problemen entgegenzutreten. Ein wesentlicher Nachteil dieser Rührtechnik stellt der hohe Energie-, Verschleiß- und Kostenaufwand dar.

In der US Patentschrift 20030141244 A1 (L. Hansen) wird ein derartiger Reaktor mit Zwischendecke - Förderschnecke und Abzugvorrichtung für die Abtrennung der Feststoffe beschrieben.

Vor allem die in den Patentschriften
- AT Patentschrift Nr. 361885 (Manahl)
- AT Patentschrift Nr. 376414 (Pfefferkorn) dokumentierten Verdrängersysteme weisen in der Praxis bei der Verarbeitung von Gärsubstraten mit geringer Neigung zur Schwimmdeckenbildung sehr gute Betriebsergebnisse auf.

Aufgrund den vorgegebenen Konstruktionen stellen sich bei diesen Verdrängersystemen bei einer Zudosierung von schwimmdeckenbildenden Gärsubstraten Probleme ein, welche in der Folge zu einer reduzierten Leistung bzw bis zu einem Stillstand der Anlage führen.

Die Erfindung stellt eine unmittelbare Weiterentwicklung dieser nach dem Verdrängersystem arbeitenden Anlagen dar und ermöglicht vor allem auch eine Energiegewinnung aus Gärsubstraten mit starker Neigung zur Schwimmdeckenbildung. Diese verbesserte Funktionstüchtigkeit wird vorschlagsgemäß durch Einbau und Betrieb von langsam laufenden und großflächig wirkenden Rührwerken erreicht, deren Antrieb über die Nutzung der Auftriebskräfte von mit Biogasgefüllten als Mischwerkzeug ausgebildeten Gasauffanghauben erfolgt. Die Rührwerke sind dabei je nach Füllstand im Reaktor zur Gänze oder auch nur zum Teil in die Gärmasse eingetaucht.

Je nach Ausführungsart - Formgebung und Führung der als Rühr- und

Mischvorrichtung ausgebildeten Gasauffanghauben, können unterschiedliche Misch- Rühr- und Gärsubstrattransporteffekte erwirkt werden.

Eine Variante stellt dabei die Führung der Gasauffanghauben auf Umlenkrollen und Schienen. Der Transport der Gasauffanghauben erfolgt über Umlaufseile oder Umlaufketten. Mit dieser Ausführungsart können vorteilhaft großflächige Behälterformen mit geringer Befüllungshöhe ausgerüstet werden. Es kann dadurch ein großflächiger Horizontaltransport der Sink- und Schwimmschichten erreicht werden. Der vertikale Transport der Schwimm- und Sinkstoffe erfolgt jeweils an den Enden im Bereich der Umlenkrollen der Rührwerke. Der vertikale Weg erfolgt dabei durch die Anordnung von jeweils 2 Umlenkrollen, deren gegenseitige vertikale Distanz auf die Füllhöhe des Reaktors abgestimmt wird.

Eine weitere wesentlich einfachere Art stellt die Fixierung der Gasauffanghauben mit Misch- und Rührwerkzeugen auf einer mit Distanzhaltern versehenen zentralen Welle dar. In diesem Falle folgen die Gasauffanghauben einer kreisrunden Umlaufbahn. Die unterschiedlichen Effekte der Gärsubstratbewegungen werden durch die speziellen Ausbildungsarten der Misch- und Rührwerkzeuge erreicht. Die Misch- und

Rührwerkzeuge können dabei zur Gänze oder auch nur zum Teil, je nach Erfordernis beweglich mit Gelenken versehen, den Gasauffanghauben zugeordnet werden.

Die beweglichen Werkzeuge können dabei durch Nutzung der eigenen Schwerkraft in jeder Umlaufstellung der Rührwerke in der gewünschten Position gehalten werden. Durch die Anordnung zusätzlicher Gewichte kann dieser Effekt verstärkt werden.

Durch diese angeführten Maßnahmen in Abstimmung auf die jeweilige Behälterform und Einbausituation des oder der Rührwerke, können optimale Misch- und Gärsubstrattransporteffekte erreicht werden.

Diese neuartige Misch- Rühr- und Gärsubstrattransporttechnik ermöglicht einen definierten Transport des Schwimmdeckenmateriales von oben in den bodennahen Bereich und in umgekehrter Weise Sinkstoffe von unten nach oben. Weiters wird eine starke gegenläufige horizontale Strömung im Schwimmdecken- sowie Grundschlammbereich erwirkt, wodurch Ablagerungen von Sinkstoffen sowie Ansammlungen von Schwimmdeckenmaterial unterbunden werden. Diese Gärsubstratumschichtungen erfolgen aufgrund der relativ langsam laufenden Rührwerke mit sehr großen Angriffsflächen gärsubstratschonend ohne Zerstörung der methanbildenden Aggregate.

Die Gaszufuhr erfolgt dabei je nach Ausführungsvariante über ein unter dem Rührwerk angeordnetes Gassammel- und Leitsystem oder durch Sammeln und Verdichten der gewonnenen Biogase in getrennten Gasräumen und der Zufuhr über ein Gasleitsystem.

Die Größenordnung der Antriebsenergie und in der Folge der Misch- und Rührintensität wird über das Fassungsvolumen der Gasauffanghauben sowie über deren Befüllungsgrad mit spezifisch leichteren Gasen vorgegeben. Die Gasauffanghauben werden in Anzahl - Größe und Lage so situiert, dass in jeder Drehstellung des Rührwerkes das über die oder der Eintrittstelle(n) eingeblasene Gas in die Gasauffanghaube(n) einströmt.

Die Rührwerke können in verschiedenen Längen und mit verschiedenen Durchmessergrößen erstellt werden. Die Gasauffanghauben können dabei sehr vorteilhaft in der Gärmasse spezifisch ähnlich schweren Materialien, wie zB Holz, erstellt werden.

Bedingt durch die Einleitung der Auftriebskräfte an der Peripherie der Rührwerke ergibt sich, bedingt durch den ebenfalls an der Peripherie entgegenwirkende Strömungswiderstand, im Betriebszustand ein annähernder Schwebezustand der Rührwerke, wodurch ein materialschonender Betriebszustand erreicht wird.

Die Gaseinblasstellen werden je nach vorgegebenem Druck in der Regel im tieferen Einblasbereich an der Außenseite unter dem Niveau der Welle bzw im niedrigeren Einblasbereich über dem Niveau der Welle über die Enden der Rührwerke an der Innenseite der Gasauffanghauben situiert. Eine weitere Gaseinblasmöglichkeit erfolgt über das Innere der Welle mit zusätzlicher Gasaustragseinrichtung in die Gasauffanghauben.

Ein weiterer und entscheidender Vorteil stellt die doppelte Nutzung des in den Reaktoren produzierten und verdichteten Gases für Rühr- und

Mischvorgänge der Reaktoreninhalte dar. Durch speziell an und in die Reaktoren integrierten Vorrichtungen werden in der Folge durch das periodische Verdichten und Ablassen der gewonnenen Gase zusätzliche Misch- und Rührvorgänge der Reaktoreninhalte erwirkt.

Anhand von Zeichnungen werden verschiedene Ausführungsbeispiele näher erläutert.

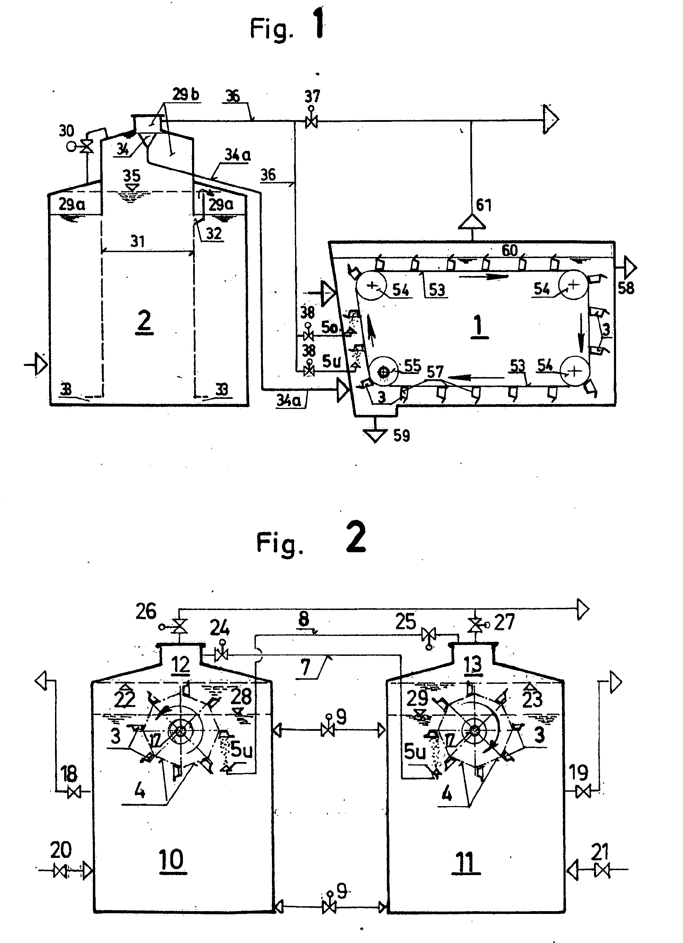

### Zu Figur 1 (Vertikalschnitt durch 2 Gasreaktoren):

Die Figur 1 stellt eine vereinfachte Darstellung von 2 Reaktoren (1) und (2) dar, in welcher Reaktor (2) gleichzeitig als Biogaserzeuger für den Rührwerksantrieb in Reaktor (1) eingesetzt ist. Die Durchmischung und Führung des Gärsubstrates in Reaktor (2) erfolgt auf hydraulischer Basis, wie folgt dargestellt.

Der Reaktor (2) ist im oberen Bereich in 2 getrennte Gassammelräume (29a) und (29b) geteilt, welche gegenseitig mit einer oder mehreren Gasüberströmungseinrichtung(en) mit Absperrorgan(en) (30) verbunden sind. Im tiefer liegenden Gärsubstratbereich wird durch den Einbau von einer oder mehreren perforierten Trennwa(ä)nd(en) (31), welche zusätzlich in Abstimmung auf das zu verarbeitende Gärsubstrat mit Leit- und Förderschächten (32 + 33) ergänzt sind, in welchen während der Verdränger- und den Mischphasen das Gärsubstrat in Bewegung gehalten wird. Die Mengenaufteilung der Gärsubstratführung wird dabei über die Strömungsquerschnitte und der speziellen Formgebung der oder den perforierten Trennw(ä)and(en) (31) sowie den Leit- und Förderschächten (32) und (33) vorgegeben. Die Verdrängerphase wird dabei über die Verriegelung der oder den Gasüberströmungsleitung(en) mit Absperrorgan(en) (30) eingeleitet. In der Folge sammelt und verdichtet sich das aus der darunter liegenden Gärmasse gewonnene - spezifisch leichtere Gas im Gassammelraum (29a). In weiterer Folge wird die darunter liegende Gärmasse volumensgleich mit der gewonnenen und verdichteten Gasmenge über die perforierte(n) Leitwa(ä)nd(e) (31) sowie die Leit- und Förderschächte) (32) und (33) in den angrenzenden Gassammelraum (29b) bis auf den durch Überlaufstelle (34) vorgegebenen max. Füllstand verdrängt. Die überschüssige Gärmasse strömt in der Folge über die Überlaufstelle (34) mit Ablaufleitung (34a) ab. Nach Erreichen des vorgegebenen Verdrängervolumens, dessen Maximum durch die tiefer liegende Öffnung des Leit- und (des) der Förderscha(ä)chte(s) (32) begrenzt wird, öffne(n)t die Gasüberströmungseinrichtung(en) mit Absperrorgan(en) (30), wodurch ein Druckausgleich in den Gassammelräumen (29a) und (29b) erwirkt wird. In der Folge strömt die während der Verdrängerphase nach oben verdrängte Gärmasse, dem Gesetz der Schwerkraft folgend, über die Öffnungen in der (den) Leitwa(ä)nd(en) (31) und über die Leit- und Förderschächte (32) und (33) soweit zurück, bis in beiden durch die Leitwand (31) getrennten Räumen annähernd derselbe Füllstand (35) erreicht ist. Durch die folgende wiederkehrende Schließung der Gasüberströmungseinrichtung(en)mit Absperrorgan(en) (30) wird die Verdrängerphase erneut eingeleitet.

In der weiteren Folge wird das verdichtete Biogas aus dem Gassammelraum (29b) über das Gasleitsystem (36) mit den Gaseinblasstellen (5u) und (5o) dem(n) Rührwerk(en) (4) im Reaktor (1) zugeführt. Das überschüssige Biogas wird dabei über eine Druckregel- und Ablasseinrichtung (37) der weiteren Verwertung zugeführt.

Bedingt durch die periodisch durchgeführten Verdränger- und Mischphasen im Reaktor (2) variiert der Gasanfall auch in Druck und Menge. Durch die Anordnung der Gaseinblasstellen (5u) und (5o) in verschiedenen Einblastiefen wird dieser variablen Gasabflusscharakteristik aus Reaktor (2) Rechnung getragen. In der Phase des Anfalles der großen Gasmengen mit erhöhtem Druck, welche mit jeder Mischphase in Reaktor (2) eingeleitet wird, kann die Gaseinspeisung auch über die tiefer liegende Gaseinblasstelle (5u) eingeleitet werden, wodurch eine zeitlich begrenzte Intensivierung des Rühr- und Mischvorganges im Reaktor (1) erreicht wird.

Nach dem durch die erhöhte Gasabnahme wiederkehrenden Druckabfall kann die Gaseinblasung nur noch die höher situierte Gaseinblasstelle (5o) mit der geringeren Einblastiefe betrieben werden.

Durch die in das Gasleitsystem (36) eingebauten Druckregel- und Ablasseinrichtungen (37) und (38) können zusätzlich Mischintensität und Mischzeiten der oder des Rührwerke(s) (57) variabel eingestellt werden.

Bei dem dargestellten Rührwerk (57) werden die als Gärsubstratmisch- und Transportwerkzeuge ausgebildeten Gasauffanghauben (3) an 2 Umlaufketten (53) mit Umlenkräder (54) geführt. Damit ein paralleler Lauf der 2 Umlaufketten (53) sichergestellt ist, werden die beiden Umlenkräder (55) über eine starre Verbindungsachse gegenseitig fixiert. Die Umlenkräder (54) und (55) werden dabei an den Endbereichen der Gassammelhauben (3) situiert und gelagert. Umlenkräder (54) und (55) laufen dabei verzahnt mit den Umlaufketten (53). Durch eine zusätzliche Führung der Gasauffanghauben (3) in oder auf Leitschienen, kann der Lauf weiter stabilisiert werden. Die Ausbildung der Gasauffanghauben (3) als Gärsubstratmisch- und Transportwerkzeug gewährleistet neben dem horizontalen Transport der Sink- und Schwimmstoffe gleichzeitig auch den vertikalen Transport derselben Stoffe von oben nach unten sowie in umgekehrter Weise von unten nach oben. Die überschüssigen Schwimmstoffe werden über deren Abzugsvorrichtung (58) sowie die mineralischen Sinkstoffe über die Abzugsvorrichtung (59) aus dem Reaktor (1) abgelassen. Das gewonnene Biogas wird im Gasraum (60) gesammelt und über die Ableitung (61) der weiteren Verwertung zugeführt.

Die Fig. 1 dargestellte Verfahrenstechnik ermöglicht die definierte Führung und Durchmischung des Gärsubstrates in den Reaktoren (1) und (2) ohne Fremdenergie sowie die volle Funktionstüchtigkeit auch beim Betrieb der Anlage mit hoher Belastung und hohem Trockensubstanzgehalt im Gärsubstrat.

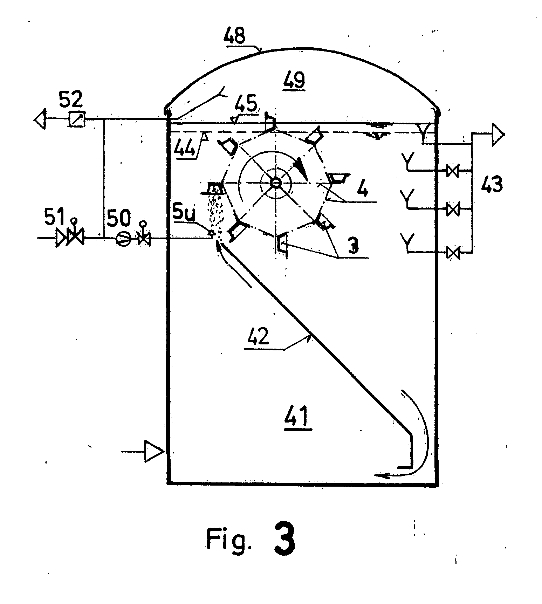

### Zu Figur 2 (Vertikalschnitt durch 2 Gasreaktoren):

Diese Schemazeichnung stellt zwei gleichartig konstruierte Reaktoren dar, welche im Gasbereich über Gasleitungen (7) und (8) sowie im Bereich der Gärsubstrate über eine oder mehrere Leitung(en) mit Absperrorgan(en) (9) gegenseitig verbunden sind. Die Reaktoren (10) und (11) sind weiters mit Rührwerken (4) ausgerüstet. Die Durchmischung der Reaktoren (10) und (11) erfolgt im Wechseltakt, vor allem durch Nutzung der im Zuge der Entspannung der gewonnenen und verdichteten Gasmengen frei werdenden Energie sowie der Nutzung der Strömungsenergie während der Verdränger- und Rückströmphase der Gärmasse zwischen den Reaktoren (10) und (11) über die Leitung(en) mit Absperrorgan(en) (9). Die Mischintervalle verlaufen wie folgt.

Die Absperrorgane (24) - (27) und Ablaufleitung mit Absperrorgan (19) sind geöffnet, alle restlichen Absperrorgane bleiben geschlossen. In der Folge sammelt und verdichtet sich das im Reaktor (10) gewonnene Gas im Gassammelraum (12) und strömt nach Erreichen des erforderlichen Gasdruckes über die Gasleitung (7) mit Gaseinblasstelle (5u) in die Gasauffanghauben (3) des Rührwerkes (4) im Reaktor (11), wodurch, bedingt durch die eintretenden Auftriebskräfte, in der Folge das Rührwerk (4) in Betrieb gesetzt wird und das im Reaktor (11) gewonnene Gas strömt gemeinsam mit dem eingeblasenen Gas über Gasabgangseinrichtung (27) zur weiteren Verwertung ab. Nach Erreichen der vorgegebenen Mischzeit im Reaktor (11) werden die Leitung(en) mit Absperrorgan(en) (9) geöffnet, wodurch in der Folge, bedingt durch den erhöhten Gasdruck, Gärsubstrat von Reaktor (10) in den Reaktor (11) verdrängt wird. Diese Verdrängung von Gärsubstrat erwirkt eine weitere Durchmischung des Gärsubstrates im Reaktor (11). Das überschüssige Gärsubstrat strömt dabei über die den max. Füllstand begrenzende Ablaufleitung mit Absperrorgan (19) ab. Nach Erreichen des vorgegebenen max. gedrückten Füllstandes (28) in Reaktor (10) wird die Gasabgangeinrichtung (26) aus Reaktor (10) geöffnet, wodurch ein Druckausgleich zwischen beiden Reaktoren erreicht wird und in der Folge die verdrängte Gärmasse dem Gesetz der Schwerkraft folgend über die Leitung(en) mit Absperrorgan(en) (9) in den Reaktor (10) zurückströmt und dessen Inhalt durchmischt. Diese Rückströmphase kann vorteilhaft als Beschickung der Reaktoren (10) und (11) mit frischer Gärmasse genutzt werden. Nach Beendigung der Rückströmphase werden die Absperrorgane (25) und Ablaufleitung mit Absperrorgan (18) geöffnet und gleichzeitig die Absperrorgane (24) - (27), die Ablaufleitung mit Absperrorgan (19) und Leitung(en) mit Absperrorgan(en) (9) geschlossen, wodurch der alternatierende Mischvorgang in umgekehrter Weise eingeleitet wird. Die Mischvorgänge werden durch die jeweilige Einstellung der Absperrorgane, wie beschrieben, im Wechseltakt zwischen 2 oder auch mehreren Reaktoren durchgeführt. Die max. gedrückten Füllstände (28) und (29) werden durch die an oberster Stelle angeordneten Leitung(en) mit Absperrorgan(en) (9) begrenzt.

Die Vorteile der unter Fig. 2 angeführten Ausbauvariante liegen vor allem in Kombination der Durchmischungsarten mit dem gasbetriebenen Rührwerk (4) und der rein auf hydraulischer Basis verlaufenden Durchmischung während der Verdränger- und Rückströmphase der Gärmasse über die Leitung(en) mit Absperrorgan(en) (9). Weiters wird durch die spezielle Anordnung von 2 oder mehreren Leitung(en) mit Absperrorgan(en) (9) die Gärsubstratführungs- und Mischintensität in den Reaktoren (10) und (11) entscheidend verbessert.

### Zu Figur 3 (Vertikalschnitt durch einen Gasreaktor):

Diese Schemazeichnung stellt einen Reaktor (41) dar, dessen Rührwerke (4) über ein darunter angeordnetes Gassammel- und Leitsystem (42) mit Biogas gespeist wird. Gleichzeitig wird die durch den Betrieb des (der) Rührwerke(s) (4) erzeugte Strömung über die Gassammel- und Leitsystem (42) bis in die Nähe der Bodenplatte des Reaktors (41) geleitet, wodurch in der Folge auch das tiefer liegende Gärsubstrat gemischt und umgewälzt wird.

Weiters ermöglicht die Gärsubstratablaufvorrichtung (43) eine Entnahme von überschüssigen Gärsubstraten in unterschiedlichen Höhen durch die in der Höhe versetzten Entnahmestellen, wobei bei einer Entnahme über die oberste Abzugsvorrichtung ohne Absperrorgan sich der max. Füllstand (45) und beim Abzug über die unteren mit Absperrorganen versehenen Vorrichtungen sich der gesenkte Füllstand (44) einstellt. Soweit die eigene über das Gassammel- und Gasleitsystem (42) geführte Gasproduktion für den Betrieb des (der) Rührwerkes(e) (4) nicht ausreicht, kann über ein Druckerhöhungsgebläse (50) oder auch von extern über die Regelstation (51) Gas dem(den) Rührwerk(en) (4) zugeführt werden. Das gewonnene Biogas wird über die Gasregelstation (52) der weiteren Verwertung zugeführt.

Der Vorteil des in Fig. 4 dargestellten Reaktors liegt vor allem in der einfachen Konstruktion, wodurch die Voraussetzungen für eine kostengünstige Erstellung gegeben sind.

## Patentansprüche

1. Verfahren zur Gewinnung von Methangas aus organisch beladenen Substraten in gelöster und fester Form, bestehend aus einem oder mehreren Behältern mit mindestens je einem Ab- und einem Zulauf versehen, **dadurch gekennzeichnet, dass** die Reaktoren (1) - (10) - (11) und (41) mit einem oder mehreren Rührwerk(en) (4) - (57) versehen sind, deren Antriebsenergie über Nutzung der Auftriebskräfte der mit in Reaktoren (2) - (10 - (11) und (41) gewonnenem Biogas gefüllten Gasauffanghauben (3) erfolgt.

2. Verfahren nach Anspruch 1 **dadurch gekennzeichnet, dass** das für den Antrieb der oder des Rührwerke(s) (4) und (57) benötigte Gas in einem oder mehreren mit einer oder mehreren Trennwa(ä)nd(en) (31) mit Leit- und Förderschächten (32) und (33) sowie einer oder mehreren Gas-überströmungseinrichtung(en) (30) ausgerüsteten Reaktor(en) (2) gewonnen und verdichtet wird und in der weiteren Folge über ein oder mehrere Gasleitsystem(e) (36) zugeführt wird.

3. Verfahren nach Anspruch 1 **dadurch gekennzeichnet, dass** 2 oder mehrere mit Rührwerken (4) oder/und (57) ausgestattete Reaktoren (10) und (11) gegenseitig mit Gasleitungen (7) und (8) samt Absperrvorrichtungen (24) und (25) für den Gastransport sowie mit einer oder mehreren Leitung(en) mit Absperrorganen (9) für den Gärsubstrattransport ausgestattet sind.

4. Verfahren nach Anspruch 1 **dadurch gekennzeichnet, dass** die Gasauffanghauben (3) durch spezielle Formgebung als Misch- und Rührwerkzeug ausgebildet sind und über Distanzhalter auf einer gemeinsamen Welle (17) geführt werden und in Form und Anzahl so gestaltet sind, dass das über die Gaseinblasstellen (5o) und (5u) eingetragene Gas in jeder Position des oder der Rührwerke(s) (4) in die Gasauffanghauben (3) einströmt.

5. Verfahren nach Anspruch 1 - 2 - 3 und 4 **dadurch gekennzeichnet, dass** das für den Betrieb des oder der Rührwerke(s) (4) benötigte Gas aus einem oder mehreren parallel oder in Reihe betriebenen Reaktor(en) (2) - (10) - (11) und (41) bezogen wird und je nach vorgegebenen Druckverhältnissen über eine oder mehrere in der Höhenlage unterschiedlich angeordnete Gaseinblasstellen (5u)-(5o) den Gasauffanghauben (3) zugeführt wird.

6. Verfahren nach Anspruch 1 - 2 - 4 und 5 **dadurch gekennzeichnet, dass** die Umlaufgeschwindigkeit der Gassammelhauben (3) über die Mengenregelung der Gaszufuhr mittels der Druckregel- und Ablasseinrichtungen (37) und (38) erfolgt.

7. Verfahren nach Anspruch 1 - 2 und 3 **dadurch gekennzeichnet, dass** das überschüssige Gärsubstrat über eine Ablaufeinrichtung (43) mit 2 oder mehreren in der Höhe versetzten Ablaufstellen versehen ist, wobei der oberste Ablauf ohne Absperrvorrichtung den max. Füllstand (45) und das tiefer liegende zur Atmosphäre offene Ablaufrohr den mind. Füllstand (44) begrenzt sowie die tiefer liegenden Entnahmestellen mit Absperrvorrichtungen ausgerüstet werden.

8. Verfahren nach Anspruch 1 - 2 - 3 - 4 - 5 und 6 **dadurch gekennzeichnet, dass** die für den Betrieb von einem oder mehreren der Rührwerk(en) (4) erforderliche Gaszufuhr über die Lagerwelle (17) erfolgt und über weitere Gasleiteinrichtungen den Gasauffanghauben (3) zugeführt wird.

9. Verfahren nach Anspruch 1 - 2 - 3 - 5 - 6 und 8 **dadurch gekennzeichnet, dass** das für den Antrieb der Rührwerke (4) und (57) benötigte Biogas über eine Druckerhöhungsstation (50) oder und (51) erfolgt.

10. Verfahren nach Anspruch 1 - 2 - 3 - 5 - 6 und 8 **dadurch gekennzeichnet, dass** mit dem für den Antrieb der Rührwerke (4) und (57) benötigten Biogas auch gleichzeitig den microbiologischen Ab- und Umbauprozess fördernde Medien der Gärmasse zugeführt werden.
